Europäisches Patentamt

European Patent Office

Office européen des brevets

(11) Numéro de publication: **0 362 020**
**A1**

## (12) DEMANDE DE BREVET EUROPEEN

(21) Numéro de dépôt: **89402552.7**

(51) Int. Cl.⁵: **B65D 77/20 , A61L 2/26**

(22) Date de dépôt: **18.09.89**

(30) Priorité: **21.09.88 FR 8812339**

(43) Date de publication de la demande:
**04.04.90 Bulletin 90/14**

(84) Etats contractants désignés:
**BE CH DE ES GB IT LI LU NL**

(71) Demandeur: **FEREMBAL**
**6, Boulevard du Général Leclerc**
**F-92115 Clichy(FR)**

(72) Inventeur: **Pelletier, Yves**
**3 Allée du Parc**
**F-54500 Vandoeuvre(FR)**
Inventeur: **Othenin, Bernard**
**3 Allée Maurice Barrès Fleville**
**F-54710 Ludres(FR)**

(74) Mandataire: **Phélip, Bruno et al**
**c/o Cabinet Harlé & Phélip 21, rue de La**
**Rochefoucauld**
**F-75009 Paris(FR)**

(54) **Conteneur destiné à recevoir des substances à stériliser et son procédé d'obtention.**

(57) L'invention a pour objet un conteneur destiné à recevoir des substances devant subir un traitement de stérilisation après le remplissage et la fermeture dudit conteneur, ce conteneur comprend au moins un fond 7 relié à un corps métallique rigide 1 et il est fermé sur la partie opposée au fond par un opercule 5 souple appliqué sur le bord 2 du corps métallique rigide 1 par l'intermédiaire d'au moins un produit de thermoscellage 4.

Fig. 1

EP 0 362 020 A1

## Conteneur destiné à recevoir des substances à stériliser et son procédé d'obtention

La présente invention concerne un conteneur destiné à recevoir des substances devant subir un traitement de stérilisation après son remplissage et sa fermeture. Elle concerne aussi son procédé d'obtention.

L'invention s'applique plus particulièrement aux boîtes destinées à recevoir des denrées alimentaires impliquant un traitement thermique de stérilisation.

Selon l'art antérieur, les boîtes métalliques rigides pour divers produits, en particulier, les denrées alimentaires y compris celles qui subissent un traitement thermique sont habituellement fermées par un couvercle, au moyen d'un double sertissage mécanique assurant un assemblage étanche.

Ces boîtes métalliques rigides sont constituées d'un corps en une ou deux parties, réalisé dans un matériau à base d'acier étamé ou chromé généralement verni ou d'un alliage d'aluminium également verni. Ce matériau métallique a une épaisseur généralement comprise entre 0,14 et 0,35 mm.

Lorsque le corps de boîte est en une partie, il est réalisé par une ou plusieurs opérations d'emboutissage complétées par diverses étapes de finition permettant d'obtenir le profil nécessaire ainsi qu'un bord dimensionnellement régulier. Avec le couvercle destiné à être serti après remplissage, cette boîte est dite en deux pièces.

Lorsque le corps de boîte est en deux parties, il est constitué d'un cylindre, soudé latéralement au moyen de l'une des techniques connues dont le soudage électrique par recouvrement, et d'un fond associé au corps de boîte par sertissage avec incorporation d'un joint élastomère appliqué préalablement sur ce fond. Avec le couvercle destiné à être serti après remplissage de la boîte, cette dernière est dite en trois pièces.

Le couvercle d'une boîte métallique fermée par sertissage, tel que décrit, ci-dessus possède l'étanchéité et la résistance nécessaire.

Cependant il ne peut être ouvert qu'à l'aide d'un outil manuel ou mécanisé ce qui constitue pour le consommateur une opération jugée fastidieuse.

Un tel couvercle serti a fait l'objet de nombreuses améliorations pour le rendre plus facile à ouvrir en incorporant différents dispositifs comme celui comportant un amincissement du métal ou incision, un rivet réalisé à partir du métal de ce couvercle et une languette fixée par ce rivet servant de levier pour perforer et ouvrir ce couvercle.

De tels couvercles dits à ouverture facile sont commercialisés depuis de nombreuses années et donnent généralement satisfaction aux consommateurs.

Ils présentent l'inconvénient de nécessiter des équipements très importants et très coûteux pour réaliser les dispositifs décrits à savoir incision, rivet et languette. Cette dernière augmente également le coût de ce couvercle.

Pour ces raisons, ces couvercles à ouverture facile ne sont utilisés que sur une partie des boîtes métalliques rigides actuellement sur le marché.

L'idée de base de la présente invention est de remplacer le couvercle métallique rigide par un opercule souple.

Pour autant qu'on le sache, il n'existe pas à l'heure actuelle de boîtes métalliques rigides renfermant des produits à stériliser qui sont obturées par un opercule souple.

On connaît des boîtes métalliques rigides pour conserver des produits lyophilisés sur lesquelles est appliquée une pellicule mince intermédiaire de matière en feuille métallique, mais ce conditionnement n'a pas à subir un traitement de stérilisation subséquent.

On connaît aussi des conteneurs, par exemple pour produits laitiers revêtus d'un opercule en feuille métallique mais d'une part la paroi n'est pas une paroi rigide au sens de la présente invention et d'autre part le produit conditionné n'a pas à être stérilisé par la suite.

On connaît enfin des barquettes à parois souples d'une épaisseur d'environ 10 à 18/100 de mm dans lesquelles sont déposées diverses denrées alimentaires telles que des confitures, mais là encore il n'existe pas le problème de stérilisation ultérieure.

US-A-3946872 a pour objet un emballage avec une fermeture en aluminium. Les revêtements thermoscellables sont appliqués sur toute la surface intérieure (corps et dessus). La gamme d'épaisseur prévue implique des corps d'emballage minces ou semi-rigides car la variation d'épaisseur se situe entre 0,025 et 0,30 mm . Le pelage se fait à la liaison du matériau et du vernis thermoscellant .

US-A-3954174 concerne des conteneurs à plusieurs compartiments et non pas un conteneur avec son système de fermeture . Les méthodes de stérilisation mentionnées (germicides , irradiation) ne sont pas celles usuellement pratiquées par l'industrie de la conserve.

DE-A-1922678 se rapporte à des emballages à base d'aluminium . Son objet est en fait la protection vis-à-vis de la corrosion. Cette protection est assurée par un revêtement de l'aluminium au moyen d'un vernis, la partie scellante étant constituée d'un film de polypropylène prévu sur le corps de l'emballage.

US-A-4197947 a pour objet un emballage pour le conditionnement de produits à usage médical . Il ne concerne pas un conteneur apte à la stérilisation par la chaleur . Il se rapporte principalement à l'aspect de la zone de scellage en tant que témoin de la bonne fermeture.

Si jusqu'à ce jour on n'a pas réalisé la fermeture d'une boîte métallique rigide par un opercule souple, la raison en est qu'il existe un problème technique sérieux pour insérer un opercule sur un emballage rigide. De nombreuses tentatives pour insérer sur une structure rigide un opercule se sont révélées négatives car les vernis du commerce notamment à base de résines vinyliques ne conviennent pas.

Dans tous les essais effectués jusqu'à ce jour, on a constaté des défauts d'étanchéité. Jusqu'à ce jour on ne pouvait pas obtenir un opercule présentant les qualités d'étanchéité nécessaire. L'opercule appliqué doit, en effet, répondre à plusieurs besoins. Il doit agir en tant que barrière aux gaz, il doit permettre un bon scellage sur les parois métalliques, un bon renforcement et une bonne rigidification lorsqu'il est mis en place. Il doit également procurer une résistance aux rayures. Toutefois la fonction la plus importante est bien entendu la fonction d'étanchéité. Il existe donc, en effet, une difficulté pour obtenir une étanchéité après stérilisation.

La différence de structure paroi rigide opercule souple implique, en outre, une difficulté technologique de thermoscellage car lorsque ces produits sont mis en contact, l'épaisseur du métal entraîne un transfert thermique perturbant un bon scellage de l'opercule.

La présente invention résout les problèmes exposés ci-dessus et prévoit d'appliquer un opercule en tout ou partie sur une boîte métallique rigide. L'opercule peut constituer alors une fenêtre qui dans le cas d'un opercule transparent permet de voir la qualité des produits conditionnés.

Bien entendu lorsque l'opercule recouvre entièrement la boîte métallique, il peut aussi être transparent.

Le consommateur peut donc apprécier sans ouverture de la boîte la qualité du contenant.

La présente invention a pour objet un conteneur destiné à recevoir des substances devant subir un traitement de stérilisation après son remplissage et sa fermeture, ledit conteneur comprenant un corps métallique rigide, et étant caractérisé en ce qu'il est fermé sur la partie opposée au fond par un opercule souple appliqué sur le bord du corps métallique rigide par l'intermédiaire d'au moins un produit de thermoscellage.

La présente invention a également pour objet un procédé d'obtention d'un conteneur destiné à recevoir les substances devant subir un traitement de stérilisation dans lequel on conforme un corps métallique rigide avec un fond, on remplit le récipient obtenu avec la substance devant recevoir un traitement ultérieur de stérilisation, on ferme ledit récipient en appliquant un opercule souple qui est solidarisé par un produit de thermoscellage prévu sur au moins le bord du corps métallique ou la surface interne de l'opercule et on achemine le conteneur ainsi fermé au poste de stérilisation.

La présente invention concerne également les caractéristiques ci-après considérées isolément ou selon toutes leurs combinaisons techniquement possibles:
- le corps métallique rigide a une épaisseur comprise dans la gamme de 0,14 à 0,35 mm, notamment de 0,15 à 0,25 mm;
- le produit de thermoscellage est un composant pelliculaire;
- le composant pelliculaire est une pellicule de polypropylène ou à base de polypropylène;
- le produit de thermoscellage est un vernis en particulier une solution ou une dispersion dans un solvant;
- le produit de thermoscellage est appliqué au moins sur le bord du corps métallique rigide ou sur l'opercule;
- l'opercule a une épaisseur comprise dans la gamme de 20 à 130 microns;
- l'opercule renferme au moins une couche barrière aux gaz;
- l'opercule est un produit souple entièrement en matière synthétique;
- l'opercule est un produit souple à base d'un complexe de matière synthétique et de métal;
- la matière synthétique est un polymère à base de polypropylène et le métal est une feuille d'alliage d'aluminium ou d'acier.

Un tel opercule dont l'épaisseur totale se situe entre 20 et 130 microns peut être obtenu selon les caractéristiques requises pour chaque application sous forme d'un matériau multicouches coextrudé et/ou contrecollé et/ou enduit.

Si nécessaire, une couche au moins est constituée d'un matériau assurant une fonction barrière. Celui-ci peut être soit métallique à base d'alliage d'aluminium ou d'acier, soit polymère tel qu'un polymère d'éthylène-vinyl-alcool (EVOH) ou un polymère de chlorure de vinylidène (PVDC).

Selon l'invention, la liaison hermétique entre l'emballage rigide et l'opercule est réalisée par un procédé de thermoscellage. A cet effet, les faces à thermosceller respectivement de la boîte rigide et de l'opercule sont revêtues de matériau adequat.

De tels matériaux sont largement diffusés à l'heure actuelle. Ils sont à base de polymères à caractère thermoplastique, par exemple, et sans caractère limitatif, on peut mentionner le polyéthylène, le polypropylène et les résines vinyliques.

Le produit apte au thermoscellage peut être appliqué de façon partielle sur les bords du récipient métallique rigide ou entièrement sur ce dernier, dans ce cas, on utilise, de préférence, une pellicule à base de polypropylène ou d'un copolymère de propylène et d'éthylène.

Ainsi, le revêtement apte au thermoscellage peut être déposé sur les faces correspondantes de la boîte et de l'opercule au cours de la fabrication de ceux-ci soit sous forme de films contre-collés ou co-extrudés soit sous forme de vernis.

Dans ce dernier cas, l'agent de scellage est préparé sous forme d'une solution ou d'une dispersion dans un solvant avec, éventuellement, des résines filmogènes. Le vernis est appliqué selon un procédé tel que l'héliogravure ou le vernissage au rouleau et est ensuite séché.

L'invention peut être mise en oeuvre de différentes façons selon le type de boîte concerné. S'il s'agit d'une boîte en deux pièces comportant un corps et un fond d'un seul tenant, l'opercule pourra être thermoscellé après remplissage de la boîte sur le bord de celle-ci et la forme aura été préparée en ménageant lors de la fabrication de la boîte une surface régulière avec un revêtement thermoscellable, cette surface étant dite bord à thermosceller.

S'il s'agit d'une boîte à trois pièces comportant un corps, un fond et un couvercle, l'opercule peut être scellé lors de la fabrication de la boîte tandis que la fermeture après remplissage s'effectue par un procédé classique, par exemple le sertissage d'un couvercle rigide.

Dans tous les cas, l'invention est combinable avec une liaison par sertissage, un fond ou couvercle classique à sertir pouvant être remplacé par une collerette annulaire rigide sur laquelle est placé un opercule thermoscellé.

Cet ensemble collerette-opercule peut être assemblé hermétiquement à la boîte par le procédé classique de sertissage. l'opercule étant ou non intégré dans le serti.

Conformément à la présente invention, l'ouverture de la boîte par le consommateur peut être obtenue soit par découpe de l'opercule à l'aide d'un couteau par exemple ce qui est rendu aisé par la faible épaisseur de cet opercule et l'appui sur la partie rigide de la boîte soit par pelage de l'opercule par traction manuelle à partir d'une languette de préhension ménagée dans l'opercule ou fixée sur celui-ci.

Divers avantages et caractéristiques de la présente invention ressortiront de la description détaillée ci-après faite en regard des dessins annexés sur lesquels:

Figure 1 est une vue de côté d'un conteneur selon l'invention.

Figure 2 est une perspective partielle du conteneur de la Figure 1.

Figure 3 illustre une variante avec un opercule revêtu d'une coiffe.

Figure 4 est un détail de la variante de la Figure 3.

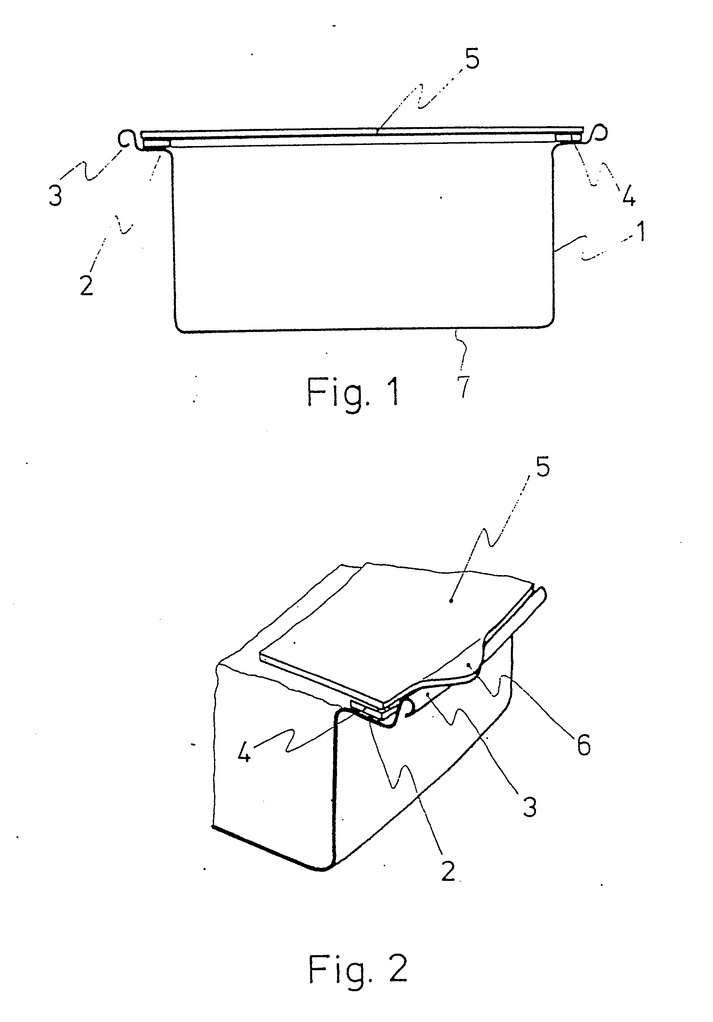

Comme représenté sur les Figures 1 et 2, à partir de feuilles de fer chromé préalablement verni sur les deux faces, on fabrique par emboutissage-réemboutissage, une boîte métallique rigide.

L'emboutissage permet de ménager à l'extrémité du corps de boîte 1, un bord annulaire 2 ayant une extrémité 3 roulée vers le bas ou dans une direction opposée, comme désiré. Le corps de boîte 1 présente aussi un fond 7.

Sur le bord annulaire 2, on a déposé un produit de thermoscellage 4. Selon une variante, on peut appliquer le produit de thermoscellage 4 dès le vernissage à plat du métal avant emboutissage.

Après remplissage de la boîte, un opercule 5 de type pelable est thermoscellé à l'aide d'une machine classique. Le thermoscellage peut être réalisé sous vide d'air compensé ou non.

L'opercule 5 est découpé de manière à laisser une languette débordante non scellée 6.

La boîte remplie et fermée est ensuite stérilisée dans un stérilisateur avec un cycle de température et de pression adapté.

Dans le mode de réalisation selon les Figures 1 et 2, l'ouverture du produit final se fait par traction manuelle à partir de la languette 6.

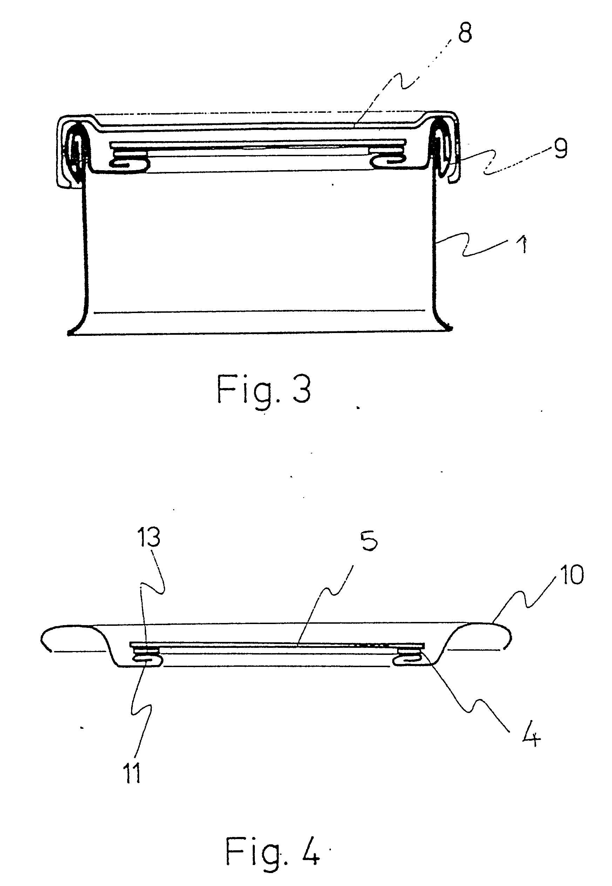

La Figure 3 représente une boîte en trois parties dont le corps cylindrique 1 est obtenu par roulage et soudage latéral d'un flan de fer blanc. Dans ce mode de réalisa tion il est prévu une capsule de surbouchage 8 recouvrant l'ensemble collerette-opercule qui a été assemblé à l'une des extrémités de la boîte par sertissage 9. Comme représenté sur la figure 4, la collerette présente pour sa partie périphérique les caractéristiques d'un fond métallique rigide pour sertissage avec notamment un bord à sertir 10 ourlé et jointé .

La partie centrale est découpée et la bordure repliée ou roulée 11 de manière à ménager un bord pour thermoscellage 13 en masquant la tranche de métal rigide.

Cette bordure peut être roulée vers sa face supérieure comme illustré sur la figure 4 ou vers sa face intérieure selon une variante non représentée.

Le bord 4 à sceller reçoit un produit de thermoscellage 4, le thermoscellage de l'opercule 5 étant effectué à l'aide d'un équipement classique.

Le conditionneur utilisant la boîte ainsi réalisée peut la fermer avec un couvercle conventionnel à sertir .

Après stérilisation, la fermeture côté opercule 5 peut être complétée par une capsule de surbouchage 8 en polyéthylène injecté ou thermoformé ou un autre matériau synthétique selon des techniques connues.

L'ouverture par le consommateur peut être réalisée comme dans l'exemple selon la Figure 1 à l'aide d'une languette ménagée dans l'opercule 5, celui-ci étant du type pelable ou par découpe avec un couteau par exemple en prenant appui sur le bord rigide de la collerette s'il s'agit d'un opercule 5 non pelable.

Les divers essais réalisés avec le produit de thermoscellage ont montré qu'on obtient un résultat satisfaisant en utilisant un vernis thermoscellant apliqué sur la boîte à base de résine époxy et de polypropylène alors que les vernis à base de polymères vinyliques ne conviennent pas.

S'agissant de l'opercule 5 des complexes non pelables satisfaisants sont constitués de polyester, d'aluminium et de polypropylène. Le polyester constitue la couche extérieure et le polypropylène la couche intérieure. Des dimensions appropriées sont de 10 à 20 microns pour la partie polyester, de 30 à 60 microns pour la partie aluminium et de 49 à 80 microns pour la partie polypropylène.

Lorsqu'on utilise un complexe pelable pour l'opercucule 5 la partie polyester constituant la couche extérieure a une épaisseur de 10 à 20 microns, la partie d'aluminium une épaisseur de 30 à 60 microns et la couche intérieure en coextrudé polypropylène une épaisseur de 40 à 80 microns. Le coextrudé est réalisé de telle manière que l'adhésion entre les couches est inférieure à celle de la liaison opercule boîte métallique.

Lors d'un essai comparatif on a constaté qu'un opercule pelable constitué d'une couche extérieure de polyester d'une épaisseur de 10 à 20 microns, d'une couche intermédiaire en aluminium d'une épaisseur de 30 à 60 microns et d'une couche interne en vernis thermoscellant pelable d'une dimension de 5 à 10 microns ne convenait pas.

Comme exemple d'opercule non pelable transparent approprié on peut mentionner un opercule constitué d'une couche externe de polyester d'une épaisseur de 10 à 20 microns, d'une couche intermédiaire en polymère de chlorure de vinylidène d'une épaisseur de 10 à 30 microns et d'une couche interne en polypropylène de 50 à 100 microns.

Les modes de réalisation illustrés sur les Figures ne constituent, bien entendu, qu'une partie des possibilités d'obtenir le conteneur selon la présente invention.

Le mode de réalisation de la présente invention peut en effet s'appliquer à divers types de boîtes métalliques rigides de forme ronde et non ronde destinées à des conditionnements de produits alimentaires faisant appel à divers procédés de traitement ou de préservation comme une stérilisation par la chaleur, une fermeture et une cuisson sous vide, une fermeture sous gaz neutre.

**Revendications**

1. Conteneur destiné à recevoir des substances devant subir un traitement de stérilisation après son remplissage et sa fermeture, ledit conteneur comprenant un corps métallique rigide (1) et étant caractérisé en ce qu'il est fermé sur la partie opposée au fond par un opercule (5) souple appliqué sur le bord (2) du corps métallique rigide par l'intermédiaire d'au moins un produit de thermoscellage (4).

2. Conteneur selon la revendication 1, caractérisé en ce que le corps métallique rigide (1) a une épaisseur comprise dans la gamme de 0,14 à 0,35 mm, notamment de 0,15 à 0,25 mm.

3. Conteneur selon l'une des revendications 1 ou 2, caractérisé en ce que le produit de thermoscellage (4) est un composant pelliculaire.

4. Conteneur selon la revendication 3, caractérisé en ce que le composant pelliculaire est une pellicule de polypropylène ou d'un copolymère à base de propylène.

5. Conteneur selon l'une quelconque des revendications 1 à 3, caractérisé en ce que le produit de thermoscellage (4) est un vernis, notamment une solution ou une dispersion dans un solvant.

6. Conteneur selon l'une quelconque des revendications 1 à 5, caractérisé en ce que le produit de thermoscellage (4) est appliqué au moins sur le bord (2) du corps métallique rigide (1) ou sur l'opercule (5).

7. Conteneur selon l'une quelconque des revendications 1 à 6, caractérisé en ce que l'opercule (5) a une épaisseur comprise dans la gamme de 20 à 130 microns.

8. Conteneur selon l'une quelconque des revendications 1 à 7, caractérisé en ce que l'opercule (5) renferme au moins une couche barrière aux gaz.

9. Conteneur selon l'une quelconque des revendications 1 à 8, caractérisé en ce que l'opercule (5) est un produit souple entièrement en matière synthétique.

10. Conteneur selon l'une quelconque des revendications 1 à 8, caractérisé en ce que l'opercule (5) est un produit souple à base d'un complexe de matière synthétique et de métal.

11. Conteneur selon l'une quelconque des revendications 1 à 10, caractérisé en ce que la matière synthétique est un polymère à base de polypropylène et le métal est une feuille d'alliage d'aluminium ou d'acier.

12. Procédé d'obtention d'un conteneur destiné à recevoir des substances devant subir un traitement de stérilisation selon lequel on conforme un corps métallique rigide avec un fond, on remplit le récipient obtenu avec la substance devant recevoir un traitement ultérieur de stérilisation, on ferme ledit récipient en appliquant un opercule souple qui est solidarisé par un produit de thermo-scellage prévu sur au moins le bord du corps métallique ou la surface interne de l'opercule et on achemine le conteneur ainsi fermé au poste de stérilisation.

Fig. 1

Fig. 2

Fig. 3

Fig. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,D | US-A-3 946 872 (W.G. STURM) * Figures 1-3; colonne 1, ligne 64 - colonne 2, ligne 4; colonne 4, ligne 29 - colonne 5, ligne 25; colonne 8, lignes 34-41; colonne 6, lignes 26-37 * | 1-8,10-12 | B 65 D 77/20 A 61 L 2/26 |
| Y | | 9 | |
| | --- | | |
| X,D | US-A-3 954 174 (R.G. KRAUS) * Figures 1-3; colonne 1, lignes 44-54; colonne 2, lignes 10-68 * | 1,3-6, 12 | |
| | --- | | |
| X,D | DE-A-1 922 678 (HUECK & BÜREN KG) * Figures 1,2; page 3, lignes 14-32; page 4, lignes 6-8; page 4, ligne 32 - page 5, ligne 2 * | 1-7,10, 11 | |
| | --- | | |
| Y,D | US-A-4 197 947 (S.M.H. ZAIDI) * Figures 1-4; colonne 2, lignes 20-48; revendications 4,5 * | 9 | |
| A,D | | 1,12 | |
| | ----- | | DOMAINES TECHNIQUES RECHERCHES (Int. Cl.5) B 65 D A 61 L |

Le présent rapport a été établi pour toutes les revendications

| Lieu de la recherche | Date d'achèvement de la recherche | Examinateur |
|---|---|---|
| LA HAYE | 31-10-1989 | PERNICE,C. |